(19) Europäisches Patentamt
European Patent Office
Office européen des brevets

(11) Publication number: **0 091 677**
**A1**

# (12) EUROPEAN PATENT APPLICATION

(21) Application number: 83103457.4

(22) Date of filing: 08.04.83

(51) Int. Cl.³: C 07 C 102/06
C 07 C 103/34

(30) Priority: 12.04.82 US 367196

(43) Date of publication of application:
19.10.83 Bulletin 83/42

(84) Designated Contracting States:
AT BE CH DE FR GB IT LI NL

(71) Applicant: STAUFFER CHEMICAL COMPANY
Westport Connecticut 06880(US)

(72) Inventor: Chan, Jimmy Hau-Hin
5331 Alhambra Valley Road
Martinez California 90553(US)

(72) Inventor: Wilson, Angus
921 Jeffrey Lane
Walnut Creek, California 94598(US)

(74) Representative: Jaeger, Klaus, Dr. et al,
JAEGER & PARTNER Patentanwälte Bergstrasse 48 1/2
D-8035 München-Gauting(DE)

## (54) Process for the production of alpha-halo-N,N-dimethylpropionamides.

(57) A process for the production of alpha-halo-N,N-dimethylpropionamides, preferably those which are optically active, comprising reacting the methyl or ethyl ester of an alpha-halopropionic acid with dimethylamine in an aqueous solution, with removal of the lower alkanol formed during the reaction.

EP 0 091 677 A1

Croydon Printing Company Ltd.

# PROCESS FOR THE PRODUCTION OF ALPHA-HALO-N,N-DIMETHYLPROPIONAMIDES

## Background and Prior Art

This invention relates to a process for the production of alpha-halo-N,N-dimethylpropionamides by reaction of an ester as defined below with N,N-dimethylamine according to the reaction

$$CH_3\overset{X}{\overset{|}{C}}H\text{-}COOR \;+\; (CH_3)_2\,NH \longrightarrow CH_3\text{-}\overset{X}{\overset{|}{C}}HCON(CH_3)_2 + ROH$$

in which X is chloro or bromo and R is methyl or ethyl.

In a preferred embodiment, the product of the process of this invention is an optical isomer of such an amide, and the ester which is also utilized is an optical isomer. The preferred ester reactant is L-methyl-2-chloropropionate. Optionally, a bromopropionate or an ethyl ester of either chloro- or bromopropionic acid may be utilized.

## Summary of the Invention

The reaction of esters with amines to produce amides is a well known reaction, commonly carried out involving many different esters and amines. In the present process, an improvement is obtained in that the dimethylamine is utilized in aqueous solution, in a concentration of between about 30 and about 40% by weight, to effectuate this reaction. The reaction is carried out preferably at room temperature, that is from about 0 to about 30°C. but may optionally be carried out at higher temperatures, at approximately the boiling point of methanol or ethanol (depending on whether the methyl or ethyl ester is utilized) as discussed below. If the process is carried out at room temperature, the need for a low temperature dimethylamine condenser, required in the prior art, is eliminated.

A further feature of the invention is that the process is carried out with the removal of methanol or ethanol, respectively, concurrently with the reaction. This has been found to be an important feature

in the operation of the process, as there is otherwise a danger that the alcohol produced may react with the amide to produce undesirable by-products. Such a possibility is most prevalent if the methyl ester is used, as methanol could react with the amide, by displacement of the chlorine or bromine atom to produce a methylated by-product. If the reactant utilized is the ethyl ester of chloropropionic acid, displacement of the chlorine atom and the resultant amide by an ethoxy group is less likely; however, if the reactant is the ethyl ester of the bromopropionic acid, such displacement is possible. The conduct of the process according to the present invention enables the production in good yield and purity (including optical purity, if desired) of the desired alpha-halo-N,N-dimethylpropionamide.

The removal of the alcohol may be accomplished in either of two ways. In a preferred embodiment, the reaction is conducted in the presence of an aromatic hydrocarbon solvent, such as benzene, toluene, or xylene, most preferably toluene. This enables the partitioning off of the alcohol produced out of the organic phase, in which the amide-producing reaction occurs, into the aqueous phase, thus removing it as a possible reactant with the amide (which is in the organic phase) and minimizing the danger of by-product production. Alternatively, the reaction could be carried out without the use of a hydrocarbon solvent, at the reflux temperature of the alcohol (methanol or ethanol, respectively) so that the alcohol is continuously removed from the reaction system as it is formed. When a hydrocarbon solvent is used, the organic and aqueous phases are separated after the reaction and the aqueous layer containing the alcohol is removed for further processing as desired. If the reaction is on the other hand carried out at the reflux temperature of the alcohol, the alcohol is removed as overhead and further processed as desired.

In order to prevent the production of undesirable by-products by over-reaction of the amine, the amount of amine used should be preferably kept below 1.5 moles per mole of the ester, with a preferred ratio of amine to ester of approximately 1.2:1.

The following represents an example of the conduct of the process according to this invention.

(a) In a flask were mixed 61.3 grams (g.) (0.50 mole) L-methyl-2chloropropionate, 100 milliliters (ml.) toluene, and 30 ml. water. There was then added dropwise a 40% by weight aqueous solution of N,N-dimethylamine, containing 67.5 g. (0.60 mole) of the amine. The reaction temperature was controlled at between about 0 and about 5°C.

The addition of the amine was carried out over a period of about 1.5 hours, with a further 30 ml. of water being added to the reaction mixture during the addition, after about 1 hour. The reaction temperature rose to about 11°C. during the addition. The mixture was stirred for about 1.5 hours after the addition was complete, during which time the reaction temperature further rose to about 19°C.

The reaction product was analyzed by gas chromatography and was determined to contain 85.7 area percent of the desired product, alpha-chloro-N,N-dimethylpropionamide.

(b) To determine its optical purity, the product was reacted with alpha-naphthol and the optical rotation of the resulting naphthoxyamide measured, as follows.

In a flask were placed 28.8 g. (0.20 mole) alpha-naphthol and 9.0 g. solid sodium hydroxide (97% pure). The mixture was stirred and heated. Then, 200 ml. toluene was added and the mixture allowed to cool to about 25°C.

Next, 25.5 g. (0.14 mole) of the alpha-chloro-N,N-dimethylpropionamide prepared in step (a) above, was added dropwise, followed by heating the mixture to reflux temperature (about 104°C), driving off water and toluene. The mixture was cooled to about 20°C., stirred overnight, then heated again to reflux (about 97°C.). The resulting product was cooled to 75°C., filtered and washed several times with warm water until the wash water pH was about 7. The organic layer was separated, and the solvent evaporated, yielding 13.4 g. (30% of theoretical) of a

solid, which, after recrystallization from ethanol, melted at 142.5°C - 143.5°C. It was identified as alpha-naphthoxy-N,N-dimethylpropionamide by spectroscopic techniques.

The product was determined to have an optical rotation of -162°, which by comparison with known standards, indicates it consists of 95+ % of the D-(-)-isomer. The recrystallization was performed so as to return both the D-(-)- and L-(+)- forms of the alpha-naphthoxypropionamide, therefore the optical rotation indicates the product of step (a) would also have been 95+ % D-(-) isomer of the chloropropionamide.

WHAT IS CLAIMED IS:

1. A method for production of an alpha-halo-N,N-dimethylpropionamide comprising reacting an ester having the formula

$$\begin{array}{c} X \\ | \\ CH_3CH\text{-}COOR \end{array}$$

in which X is chlorine or bromine and R is methyl or ethyl with an aqueous solution of dimethylamine, and continuously removing evolved alcohol having the formula ROH from the reaction mixture.

2. A method according to Claim 1 in which X is chlorine and R is methyl.

3. A method according to Claim 1 in which the aqueous solution contains between about 30% and about 40% of the amine by weight.

4. A method according to Claim 1 conducted in the presence of an aromatic hydrocarbon solvent.

5. A method according to Claim 4 in which the solvent is toluene.

6. A method according to Claim 1 in which the reaction is conducted at a temperature of between about 0° and about 25°C.

7. A method according to Claim 1 in which the amine is utilized in a molar ratio with respect to the ester of between about 1 and about 1.5.

8. A method according to Claim 1 conducted at a temperature of about the boiling point of the alcohol.

9. A method according to Claim 1 in which the reactant ester and product amide are optically active.

0091677

European Patent Office

# EUROPEAN SEARCH REPORT

Application number

EP 83 10 3457

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | US-A-3 342 862 (W.J. BOARD et al.) * Claims * | 1-9 | C 07 C 102/06<br>C 07 C 103/34 |
| Y | FR-A-1 372 964 (BASF) * Abstract * | 1-9 | |
| Y | BE-A- 667 686 (MONSANTO) * Abstract * | 1-9 | |
| P,Y | EP-A-0 072 884 (STAUFFER) * Claims * | 1-9 | |
| | | | TECHNICAL FIELDS SEARCHED (Int. Cl. 3) |
| | | | C 07 C 102/00<br>C 07 C 103/00 |

The present search report has been drawn up for all claims

| Place of search | Date of completion of the search | Examiner |
|---|---|---|
| THE HAGUE | 07-07-1983 | MOREAU J.M. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82